# EUROPEAN PATENT APPLICATION

(11) **EP 1 632 469 A1**
(43) Date of publication of application: **08.03.2006**
(21) Application number: 04745777.5
(22) Date of filing: 04.06.2004
(51) Int. Cl.: C07C 67/10, C07C 69/54

(54) **PROCESS FOR PRODUCING CARBOXY-TERMINATED LACTONE POLYESTER/UNSATURATED MONOMER**

(30) Priority: 06.06.2003 JP 2003194609
(71) Applicant: DAICEL CHEMICAL INDUSTRIES, LTD., Sakai-shi, Osaka 590-0905 (JP)
(72) Inventor: NAKAMOTO, Masanobu, Iwakuni-shi, Yamaguchi 741-0061 (JP); ITO, Masaaki, Ohtake-shi, Hiroshima 739-0651 (JP)
(74) Representative: Grünecker, Kinkeldey, Stockmair & Schwanhäusser Anwaltssozietät
(86) International application number: PCT/JP2004/008175
(87) International publication number: WO 2004/108650

(57) **Abstract**

A method produces an unsaturated lactone-derived polyester monomer of Structural Formula (1): 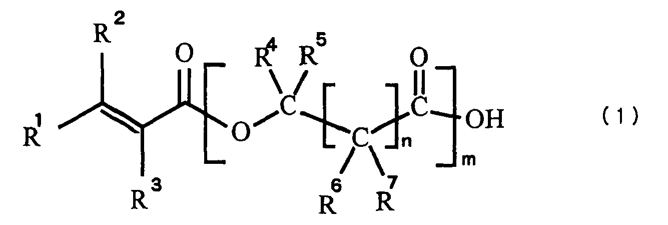
wherein each of R¹, R², R³, R⁴, R⁵, R⁶, and R⁷ represents a substituent selected from hydrogen atom, a substituted or unsubstituted alkyl group having one to ten carbon atoms, a substituted or unsubstituted aryl group, a substituted or unsubstituted alkoxycarbonyl group, a substituted or unsubstituted alkoxy group, a substituted or unsubstituted aryloxy group, and a halogen atom; n denotes an integer from 1 to 7; and m denotes an integer from 1 to 100, by reacting a carboxyl-containing radically polymerizable unsaturated monomer with a cyclic lactone using an acidic catalyst at normal pressure in the presence of 1 to 50 parts by weight of water to 100 parts by weight of the total of the carboxyl-containing radically polymerizable unsaturated monomer and the cyclic lactone; and carrying out dehydration under reduced pressure for removing low boiling components to form an ester bond between a by-produced water-initiated lactone oligomer and the carboxyl-containing radically polymerizable unsaturated monomer to thereby reduce a hydroxyl value to 5.0 mg KOH/g or less. Unsaturated lactone-derived polyester monomers having a terminal carboxyl group with practical quality can be produced at low cost.

## Description

### Technical Field

The present invention relates to a method for producing an unsaturated lactone-derived polyester monomer having a terminal carboxyl group.

### Background Art

Highly functional materials such as photo-curable resist materials, thermosetting coating materials, adhesives, bridging materials; and modifiers for paper, fibers, and leather are important industrial materials generally used in various industrial fields such as automobiles, electrics, electronics, and architecture.

Radically polymerizable unsaturated monomers having a carboxyl group are widely used as important raw materials or intermediates for these highly functional materials. To obtain a highly functional material to thereby yield a product with desired properties, an optimal carboxyl-containing radically polymerizable unsaturated monomer must be selected according to the purpose.

Of these carboxyl-containing radically polymerizable unsaturated monomers, polyester unsaturated monomers having a radically polymerizable unsaturated group at one end of a molecular chain and a carboxyl group at the other end are specifically important, since a variety of structures can be introduced into the polymer chain. Demands have therefore been made on methods for economically and efficiently producing such polyester unsaturated monomers.

Conventional methods for producing a polyester unsaturated monomer having a terminal carboxyl group include a method of reacting a halide, an active ester, or an acid anhydride of an unsaturated carboxylic acid with a ω-hydroxycarboxylic acid in the presence of a base; and a method of reacting an unsaturated carboxylic acid with a ω-halogenocarboxylic acid. These methods, however, generally require expensive raw materials or require the step of removing a large quantity of by-products.

Japanese Unexamined Patent Application Publication No. 60-67446 reports a method of reacting a carboxyl-containing radically polymerizable unsaturated monomer with a cyclic lactone in the presence of an acidic catalyst. This method, however, tends to yield a product deteriorated in quality such as hue due to its thermal hysteresis and has a low production efficiency. This is because a long-chain polyester is formed from a carboxyl-containing radically polymerizable unsaturated monomer added with a large number of caprolactone molecules in early stages of the reaction and has a low reaction rate with the residual carboxyl-containing radically polymerizable unsaturated monomer, and it takes a long time to sufficiently increase the conversion of the raw material carboxyl-containing radically polymerizable unsaturated monomer to thereby yield a product with practical quality having a desired degree of lactone addition.

In contrast, an increased amount of the acidic catalyst for accelerating the reaction adversely affects the hue of the product and invites increased by-products, which in tern decreases the purity of the product.

### Disclosure of the Invention

Accordingly, an object of the present invention is to provide a method of producing an unsaturated lactone-derived polyester monomer having a terminal carboxyl group with practical quality at low cost.

After intensive investigations to achieve the above object, the present inventors have found that the conversion of a raw material carboxyl-containing radically polymerizable unsaturated monomer can be increased by reacting the carboxyl-containing radically polymerizable unsaturated monomer with a cyclic lactone by the catalysi,s of an acid catalyst in the coexistence of a specific amount of water, and that an unsaturated lactone-derived polyester monomer having a terminal carboxyl group with practical quality can be produced at low cost by further including the step of removing low boiling components under reduced pressure (reaction). The present invention has been achieved based on these findings.

The term "removal (reaction) of low boiling components under reduced pressure" herein means a reaction in which dehydration is carried out under reduced pressure for removing low boiling components; and thereby forming an ester bond between a by-produced water-initiated lactone oligomer and the carboxyl-containing radically polymerizable unsaturated monomer to reduce the water-initiated lactone oligomer to a practically trivial level, i.e., a hydroxyl value of 5.0 mg of KOH per gram or less.

Specifically, the present invention provides, in a first aspect, a method for producing an unsaturated lactone-derived polyester monomer of Structural Formula (1): 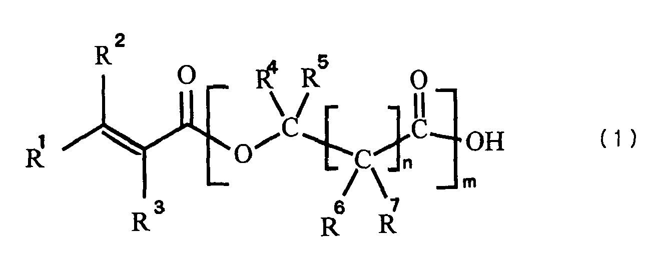
wherein each of R¹, R², R³, R⁴, R⁵, R⁶, and R⁷ represents a substituent selected from the group consisting of hydrogen atom, a substituted or unsubstituted alkyl group having one to ten carbon atoms, a substituted or unsubstituted aryl group, a substituted or unsubstituted alkoxycarbonyl group, a substituted or unsubstituted alkoxy group, a substituted or unsubstituted aryloxy group, and a halogen atom, wherein R¹, R², R³, R⁴, R⁵, R⁶, and R⁷ may be combined to form one or more rings; n denotes an integer from 1 to 7; and m denotes an integer from 1 to 100, the method comprising the steps of reacting a carboxyl-containing radically polymerizable unsaturated monomer with a cyclic lactone by the catalysis of an acidic catalyst in the presence of 1 to 50 parts by weight of water to 100 parts by weight of the total of the carboxyl-containing radically polymerizable unsaturated monomer and the cyclic lactone; and carrying out dehydration under reduced pressure for removing low boiling components to form an ester bond between a by-produced water-initiated lactone oligomer and the carboxyl-containing radically polymerizable unsaturated monomer to thereby reduce a hydroxyl value to 5.0 mg of KOH per gram or less.

The present invention provides, second, the method for producing an unsaturated lactone-derived polyester monomer of the first aspect of the present invention, in which the carboxyl-containing radically polymerizable unsaturated monomer is at least one selected from the group consisting of acrylic acid, methacrylic acid, maleic acid, and itaconic acid.

Further, the present invention provides, in a third aspect, the method for producing an unsaturated lactone-derived polyester monomer of the first or second aspect of the present invention, in which the cyclic lactone is at least one selected from the group consisting of ε-caprolactone, trimethyl-ε-caprolactone, monomethyl-ε-caprolactone, γ-butyrolactone, γ-valerolactone, and δ-valerolactone.

In addition, the present invention provides the method for producing an unsaturated lactone-derived polyester monomer of any one of the first, second, and third aspects of the present invention, further comprising carrying out dehydration under reduced pressure for removing low boiling components in the presence of residual carboxyl-containing radically polymerizable unsaturated monomer to form an ester bond between the water-initiated lactone oligomer as a by-produced to thereby reduce the hydroxyl value to 5.0 mg of KOH per gram or less.

### Brief Description of the Drawings

FIG. 1 shows a GPC peak profile of a product in Example 1.
FIG. 2 shows a ¹H-NMR spectrum of the product in Example 1.
FIG. 3 shows a GPC peak profile of a product in Example 2.
FIG. 4 shows a ¹H-NMR spectrum of the product in Example 2.
FIG. 5 shows a GPC peak profile of a product in Example 3.
FIG. 6 shows a ¹H-NMR spectrum of the product in Example 3.
FIG. 7 shows a ¹H-NMR spectrum of a product in Example 4.
FIG. 8 shows a ¹H-NMR spectrum of a product in Comparative Example 1.
FIG. 9 shows plots of the change with time in residual acrylic acid as comparison between syntheses in Example 4 and Comparative Example 1.

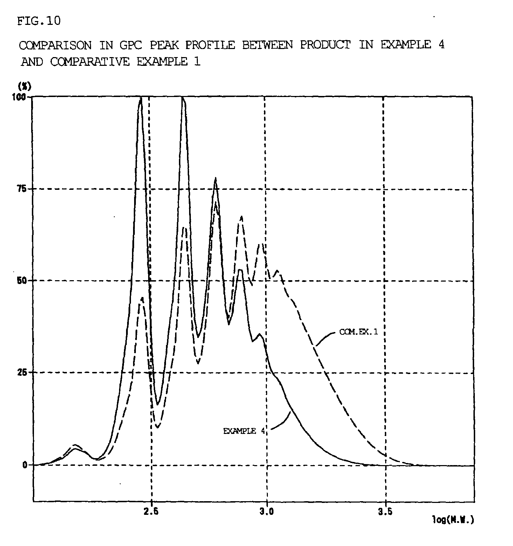

FIG. 10 shows plots of GPC peak profiles as comparison between products in Example 4 and Comparative Example 1.

### Best Mode for Carrying Out the Invention

The present invention will be illustrated in detail below. According to the present invention, an unsaturated lactone-derived polyester monomer is produced by reacting a carboxyl-containing radically polymerizable unsaturated monomer with a cyclic lactone by the catalysis of an acidic catalyst in the presence of 1 to 50 parts by weight of water to 100 parts by weight of the total of the carboxyl-containing radically polymerizable unsaturated monomer and the cyclic lactone (water-adding reaction step) ; and carrying out a reaction under reduced pressure for removing low boiling components (step (reaction) of removing low boiling components under reduced pressure). This production method is inexpensive as compared to conventional methods and can produce an unsaturated lactone-derived polyester monomer having an average repetition number m in Structural Formula (1) of 2.5 or less.

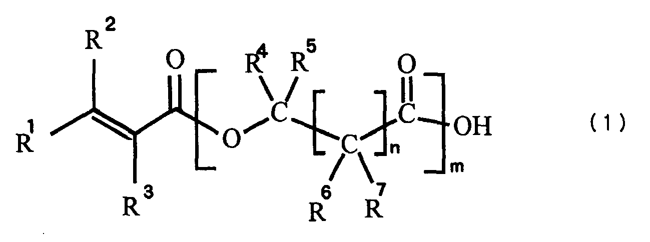

In Structural Formula (1), each of R¹, R², R³, R⁴, R⁵, R⁶, and R⁷ represents a substituent selected from the group consisting of hydrogen atom, a substituted or unsubstituted alkyl group having one to ten carbon atoms, a substituted or unsubstituted aryl group, a substituted or unsubstituted alkoxycarbonyl group, a substituted or unsubstituted alkoxy group, a substituted or unsubstituted aryloxy group, and a halogen atom, wherein R¹, R², R³, R⁴, R⁵, R⁶, and R⁷ may be combined to form one or more rings, wherein mR⁴s and mR⁵s may be independently the same as or different from each other, wherein R⁶ and R⁷ may be the same as or different from each other, and wherein mnR⁶s and mnR⁷s may be independently the same as or different from each other; n denotes an integer from 1 to 7; and m denotes an integer from 1 to 100.

An example of the carboxyl-containing radically polymerizable unsaturated monomer for use in the present invention is a compound having a structure of Formula (2): 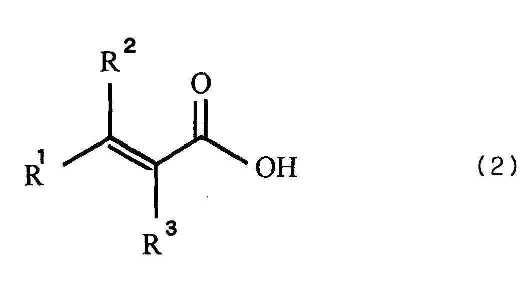
wherein each of R¹, R², and R³ represents a substituent selected from the group consisting of hydrogen atom, a substituted or unsubstituted alkyl group having one to ten carbon atoms, a substituted or unsubstituted aryl group, a substituted or unsubstituted alkoxycarbonyl group, a substituted or unsubstituted alkoxy group, and a substituted or unsubstituted aryloxy group, and wherein R¹, R², and R³ may be combined to form one or more rings.

Preferred examples of the carboxyl-containing radically polymerizable unsaturated monomer are acrylic acid, methacrylic acid, maleic acid, itaconic acid, 1-cyclohexene-1-carboxylic aid, 1-cyclopentene-1-carboxylic acid, and substituted derivatives of these, of which acrylic acid and methacrylic acid are typically preferred.

Cyclic lactones for use in the present invention include those having a structure represented by following Formula (3): 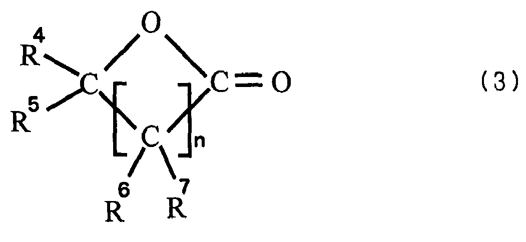
wherein each of R⁴, R⁵, R⁶, and R⁷ represents a substituent selected from the group consisting of hydrogen atom, a substituted or unsubstituted alkyl group having one to ten carbon atoms, a substituted or unsubstituted aryl group, a substituted or unsubstituted alkoxycarbonyl group, a substituted or unsubstituted alkoxy group, a substituted or unsubstituted aryloxy group, and a halogen atom, wherein R⁴, R⁵, R⁶, and R⁷ may be combined to form one or more rings, wherein R⁶ and R⁷ may be the same as or different from each other, and wherein nR⁶s and nR⁷s may be independently the same as or different from each other; n denotes an integer from 1 to 7.

Specific examples of the cyclic lactones are ε-caprolactone, trimethyl-ε-caprolactone, monomethyl-ε-caprolactone, γ-butyrolactone, γ-valerolactone, δ-valerolactone, undecanoic γ- or δ-lactone, dihydro-3-methoxy-4,4-dimethyl-2(3H)-furanone, 2-oxabicyclo[3.3.0]octanone, 2-oxabicyclo[3.3.0]oct-6-en-3-one, phthalide, and 5,5-pentamethylenetetrahydro-pyran-2-one.

Acidic catalysts for use in the reaction between a carboxyl-containing radically polymerizable unsaturated monomer and a cyclic lactone in the present invention include organotitanium compounds such as tetraethyl titanate, tetrapropyl titanate, and tetrabutyl titanate; organotin compounds such as stannous octoate, dibutyltin oxide, dibutyltin dilaurate, and mono-n-butyltin fatty acid salts; Lewis acids including stannous halides such as stannous chloride, stannous bromide, and stannous iodide; and Broensted acids such as sulfuric acid, p-toluenesulfonic acid, benzenesulfonic acid, and sulfonic acid ion exchange resins. Among them, p-toluenesulfonic acid, benzenesulfonic acid, and sulfuric acid are preferred for their high solubility in a reaction mixture and excellent handleability.

The amount of the acidic catalyst is preferably 0.1 to 20 parts by weight and more preferably 1 to 10 parts by weight to 100 parts by weight of the total of the carboxyl-containing radically polymerizable unsaturated monomer and the cyclic lactone. If the amount of the catalyst is less than 0.1 part by weight, the quality may deteriorate and the method may not be economical, since the reaction proceeds markedly slowly and the reaction time is prolonged. In contrast, if it is 20 parts by weight or more, by-products may increase to adversely affect the quality, although the reaction time is shortened.

Water for use in the reaction between the carboxyl-containing radically polymerizable unsaturated monomer and the cyclic lactone in the present invention can be added as water or be contained in a compound as water of crystallization. The amount of water is preferably 1 to 50 parts by weight and more preferably 2 to 10 parts by weight to 100 parts by weight of the total of the carboxyl-containing radically polymerizable unsaturated monomer and the cyclic lactone.

If the amount of water in the reaction is less than 1 part by weight to 100 parts by weight of the total of the carboxyl-containing radically polymerizable unsaturated monomer and the cyclic lactone, the conversion rate of the carboxyl-containing radically polymerizable unsaturated monomer is not sufficient to thereby prolong the reaction time. In contrast, if it is 50 parts by weight or more, the product is excessively hydrolyzed and the time for removal (reaction) of low boiling components under reduced pressure is prolonged.

By adjusting the amount of water in the reaction within the above-specified range, the conversion of the carboxyl-containing radically polymerizable unsaturated monomer can be increased 5% or more, preferably 10% or more, and further preferably 15% or more than the case where the amount of water is less than the above-specified range.

A suitable solvent can be used in the reaction between the carboxyl-containing radically polymerizable unsaturated monomer and the cyclic lactone. The type of the solvent is not specifically limited, as long as it does not undergo a side reaction with the product.

The amount of the cyclic lactone relative to the carboxyl-containing radically polymerizable unsaturated monomer is decided according to the molecular weight of a target unsaturated lactone-derived polyester monomer and varies depending on the amounts of water, catalyst, and solvent. The amount of the cyclic lactone is preferably 5 to 2000 parts by weight to 100 parts by weight of the carboxyl-containing radically polymerizable unsaturated monomer.

The reaction between the carboxyl-containing radically polymerizable unsaturated monomer and the cyclic lactone in the present invention is carried out in the following manner. Initially, the reaction is conducted under normal pressure or under a pressure (under a load). From the time when the conversion rate of the carboxyl-containing radically polymerizable unsaturated monomer becomes low, the pressure inside the reactor is gradually reduced to thereby distill off excessive, unreacted carboxyl-containing radically polymerizable unsaturated monomer and water under reduced pressure. Thereafter, the reaction is terminated by deactivating the catalyst at the time when dehydration condensation between a by-produced water-initiated lactone oligomer and the carboxyl-containing radically polymerizable unsaturated monomer sufficiently proceeds and the amount of the water-initiated lactone oligomer is reduced to a trivial amount.

Reaction temperatures in the water-adding reaction step and the step of removing (reaction) low boiling components are 0°C to 150°C and preferably 60°C to 120°C. If the reaction temperatures are lower than 0°C, the reaction rate may be markedly low and the reaction may not be economical. In contrast, if they are 150°C or higher, polymerization between radically polymerizable unsaturated monomer molecules significantly occur. The reaction can be carried out under the flow of air, but the oxygen concentration may be reduced to such an extent as to prevent radical polymerization of radically polymerizable unsaturated groups of the raw material and product to thereby avoid, for example, safety problems and coloring. Where necessary, polymerization inhibitors can be added in the reaction.

After the completion of the reaction, unreacted carboxyl-containing radically polymerizable unsaturated monomers are removed to thereby yield an unsaturated lactone-derived polyester monomer.

The method may further comprise a purification step after or during the removal of the carboxyl-containing radically polymerizable unsaturated monomer. Examples of the purification step are, by taking p-toluenesulfonic acid as the reaction catalyst as an example, the step of diluting a salt formed by the action of a solution containing sodium hydroxide added so as to neutralize and deactivate the catalyst, with an organic solvent such as toluene, and removing the salt typically by filtration or washing with water; and the step of decolorizing typically using activated carbon.

Unsaturated lactone-derived polyester monomers having a terminal carboxyl group produced by the method of the present invention can be advantageously used as raw materials for synthetic resins or components in compositions. Specifically, they can be used for resist ink compositions typically for semiconductors and printed boards; optical appliances such as optical filters, prisms, mirrors, and photographic materials; members for light sources such as fluorescent lamps and mercury lamps; members for precision instruments and electronic/electric equipment; barrier materials typically against electromagnetic waves generated from various displays; glass replacements and surface coating materials thereof; coating materials for window glass, lighting glass, and light source protective glass typically for houses, facilities, and transport machines; interior and exterior materials and interior and exterior coating materials typically for houses, facilities, transport machines, and automobiles; containers or packaging materials typically for foodstuffs, chemical products, and pharmaceutical preparations; sheet or film materials for agricultural or industrial use; printed matter; dyed articles; textile goods and fibers for clothing materials such as sports clothes, stockings, and headgear; household interior goods such as curtains, carpets, and hangings; optical materials such as plastic lenses and contact lenses; stationery such as tapes and inks; and signs, marking devices, and surface coating materials therefor.

### Examples

The present invention will be illustrated in further detail with reference to several examples below which by no means limit the scope of the present invention.

All percentages are by weight.

### Example 1

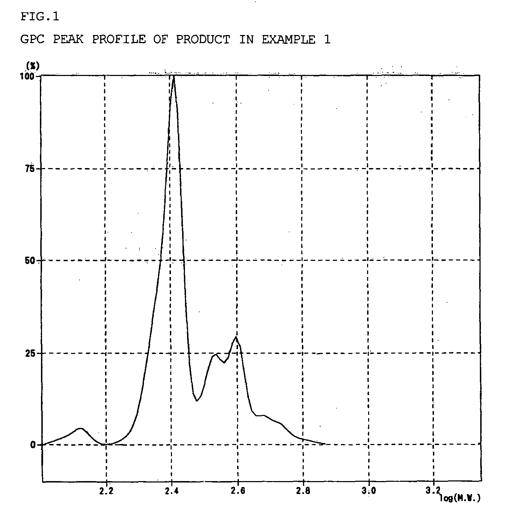

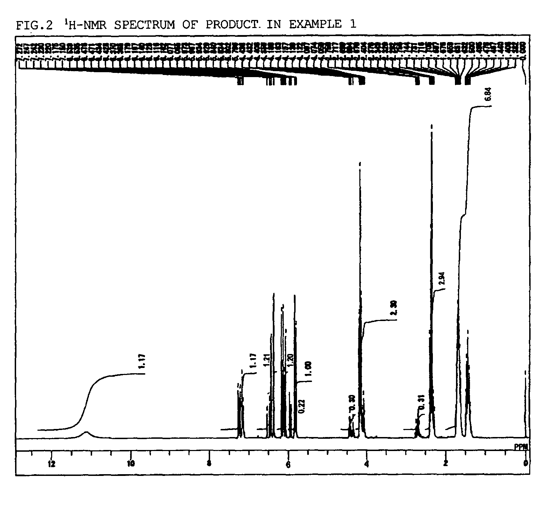

In a glass flask equipped with a cooling tube, an exhaust vent, a nitrogen inlet tube, a thermometer, and a stirrer were mixed 4002 g (55.5 mol) of acrylic acid, 0.89 g (7.2 mmol) of 4-methoxyphenol (MEHQ), 100 g (5.5 mol) of pure water, and 157 g (0.8 mol) of p-toluenesulfonic acid monohydrate. After elevating the inner temperature to 80°C, 634 g (5.6 mol) of ε-caprolactone was added over four hours, and the mixture was stirred at 80°C for further four hours. Thereafter, while holding the inner temperature to 80°C and continuing the stirring, the pressure was gradually reduced from 200 mmHg to 70 mmHg over one hour, followed by removal of low boiling components under reduced pressure at 50 mmHg for twenty minutes and at 30 mmHg to 20 mmHg for twenty minutes. At this time, a distillate was distillated in an amount about six times as much as the weight of the charged water. An analysis of the distillate by ¹H-NMR revealed that neither ε-caprolactone nor a hydrolysate thereof, 6-hydroxycaproic acid, were detected in the distillate and that the distillate was a 1:5 mixture of water and acrylic acid. After cooling to 30°C, the reaction mixture was combined with 33.04 g (0.8 mol) of NaOH as a 10% aqueous solution. The reaction mixture was then combined with 4.0 liters of toluene, washed with three portions of 2.5 liters of pure water, and the organic layer was concentrated under reduced pressure in a rotary evaporator to remove low boiling components. As a result, 1216 g of a pale yellow oily reaction product was obtained. A tetrahydrofuran eluate of the product was analyzed by GPC, and the result is shown in FIG. 1. A GPC peak profile analysis revealed that the product has a number-average molecular weight (Mn) of 269 and a weight-average molecular weight (Mw) of 290 in terms of polyethylene glycol. The ¹H-NMR spectrum of the product is shown in FIG. 2. An average number of moles of ε-caprolactone added to acrylic acid is 1.15 as calculated from the integral of the ¹H-NMR spectrum. The product has an acid value of 272.2 mg of KOH per gram and a hydroxyl value of 0.8 mg of KOH per gram as determined according to the measuring method in Japanese Industrial Standards (JIS) K 0070.

### Example 2

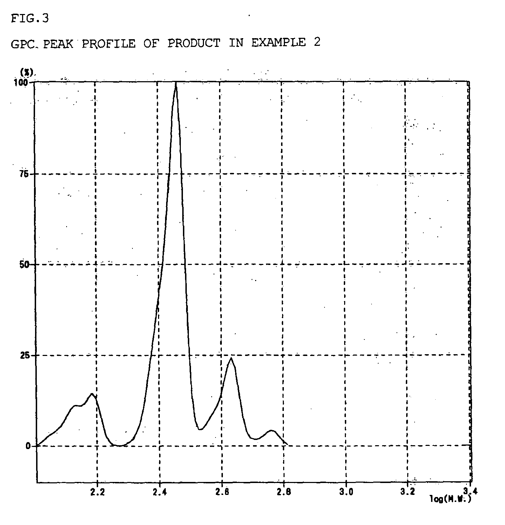

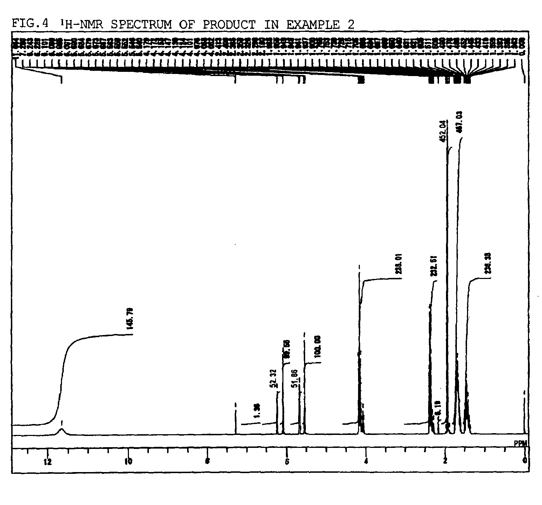

In a glass flask equipped with a cooling tube, an exhaust vent, a nitrogen inlet tube, a thermometer, and a stirrer were mixed 4779 g (55.5 mol) of methacrylic acid, 0.89 g (7.2 mmol) of MEHQ, 100 g (5.5 mol) of pure water, and 182.7 g (0.96 mol) of p-toluenesulfonic acid monohydrate. After elevating the inner temperature to 80°C, 634 g (5.5 mol) of ε-caprolactone was added over four hours, and the mixture was stirred at 80°C for further four hours. Thereafter, the temperature was once lowered to room temperature, and the pressure was gradually reduced to a range of 100 to 80 mmHg. Then, the inner temperature was gradually elevated to 80°C over 60 minutes, and the pressure was further reduced to 50 mmHg to remove low boiling components under reduced pressure for five hours to yield 1436 g of a distillate. After cooling to 30°C, the reaction mixture was combined with 38. 49 g (0.96 mol) of NaOH as a 10% aqueous solution. The reaction mixture was then combined with 4.0 liters of toluene, washed with three portions of 2.5 liters of pure water, and the organic layer was concentrated to 3410 g at 20 mmHg in a rotary evaporator. Then, 728 g of the concentrate was further concentrated at 5 mmHg in a rotary evaporator to thereby remove low boiling components, and filtrated through a glass filter. As a result, 337 g of an oily reaction product was obtained. A tetrahydrofuran eluate of the product was analyzed by GPC, and the result is shown in FIG. 3. A GPC peak profile analysis revealed that the product has a number-average molecular weight (Mn) of 270 and a weight-average molecular weight (Mw) of 295 in terms of polyethylene glycol. The ¹H-NMR spectrum of the product is shown in FIG. 4. An average number of moles of ε-caprolactone added to acrylic acid is 1.56 as calculated from the integral of the ¹H-NMR spectrum. The product has an acid value of 319.1 mg of KOH per gram and a hydroxyl value of 0.0 mg of KOH per gram.

### Example 3

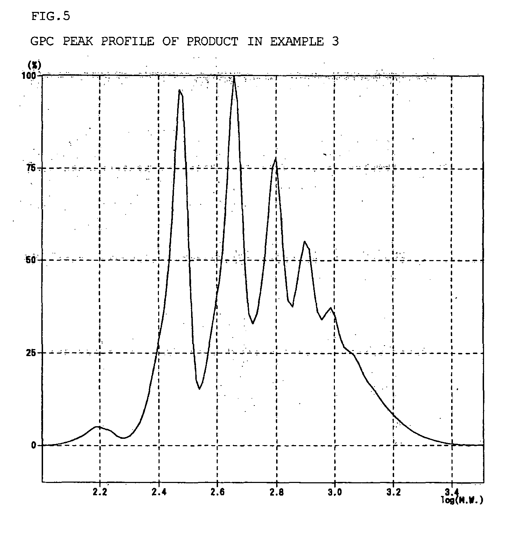

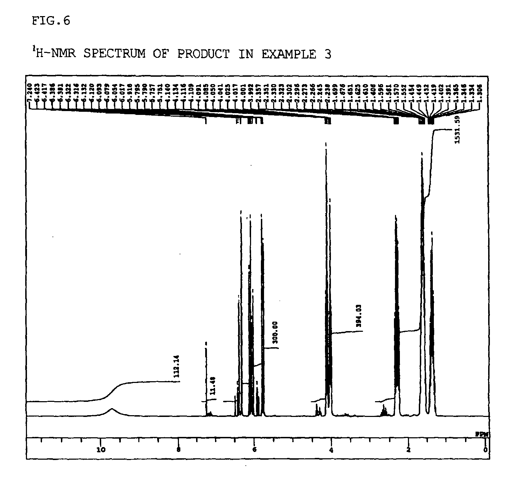

An aliquot of p-toluenesulfonic acid monohydrate (95.8 g; 0.5 mol) was added over 30 minutes to a mixture of 1200 g (16.5 mol) of acrylic acid, 1520 g (13.5 mol) of ε-caprolactone, 2.32 g (18.7 mmol) of MEHQ, and 95.8 g (0.5 mol) of pure water in a glass flask equipped with a cooling tube, an exhaust vent, a nitrogen inlet tube, a thermometer, and a stirrer. The inner temperature was elevated to 80°C, and the mixture was stirred at 80°C for four hours. Thereafter, while holding the inner temperature to 80°C and continuing the stirring, the pressure was gradually reduced from 200 mmHg to 70 mmHg over one hour and twenty minutes, followed by removal of low boiling components under reduced pressure at 50 mmHg for twenty minutes and at 30 mmHg to 20 mmHg for twenty minutes. At this time, a distillate was distilled in an amount about three times as much as the weight of the charged water. After cooling to 30°C, the reaction mixture was combined with 20.2 g (0.5 mol) of NaOH as a 10% aqueous solution. The reaction mixture was then combined with 4.0 liters of toluene, washed with three portions of 2.7 liters of pure water, and the solution was concentrated under reduced pressure in a rotary evaporator to remove low boiling components. As a result, 1964 g of an oily reaction product was obtained. The reaction product has an acid value of 198 mg of KOH per gram and a hydroxyl value of 3.0 mg of KOH per gram. It was found to have a viscosity of 115 cps (25°C) as determined with an E type viscometer and have a hue of 2 in terms of Gardner color scale. A tetrahydrofuran eluate of the product was analyzed by GPC, and the result is shown in FIG. 5. A GPC peak profile analysis revealed that the product has a number-average molecular weight (Mn) of 438, a weight-average molecular weight (Mw) of 568, and a Mw/Mn ratio of 1.30 in terms of polyethylene glycol. The ¹H-NMR spectrum of the product is shown in FIG. 6. An average number of moles of ε-caprolactone added to acrylic acid is 2.03 as calculated from the integral of the ¹H-NMR spectrum.

### Example 4

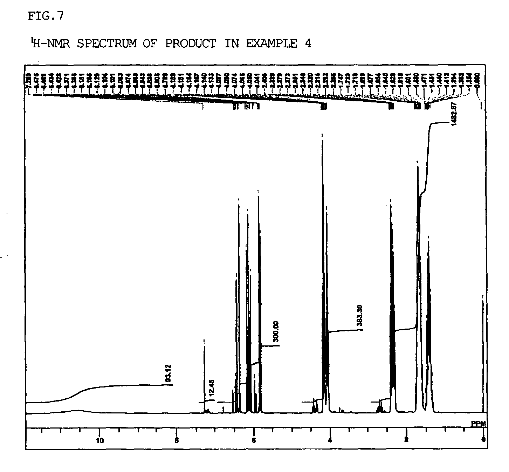

In a glass flask equipped with a cooling tube, an exhaust vent, a nitrogen inlet tube, a thermometer, and a stirrer were placed 400.0 g (5.55 mol) of acrylic acid, 28.1 g (1.56 mol) of pure water, 0.442 g (3.56 mmol) of MEHQ, and 30.1 g (0.158 mol) of p-toluenesulfonic acid monohydrate. After elevating the inner temperature to 80°C, 507.0 g (4.44 mol) of ε-caprolactone was added dropwise over four hours while stirring at 80°C. While holding the reaction temperature to 80°C with stirring, the mixture was stirred for further four hours, the pressure was gradually reduced from 200 mmHg to 70 mmHg over one hour and twenty minutes, followed by removal of low boiling components under reduced pressure at 50 mmHg for twenty minutes and at 30 mmHg to 20 mmHg for twenty minutes. At this time, a distillate was distilled in an amount about three times as much as the weight of the charged water. After cooling to 30°C, the reaction mixture was combined with 6.65 g (0.166 mol) of a 10% aqueous solution of NaOH. The reaction mixture was then combined with 1.3 liters of toluene, washed with three portions of 0. 9 liter of pure water, and the solution was concentrated under reduced pressure in a rotary evaporator to remove low boiling components. As a result, 1678.1 g of an oily reaction product was obtained. A ¹H-NMR analysis revealed that 2.03 moles on average of ε-caprolactone was added to 1 mole of acrylic acid, and that 36.8% of the charged acrylic acid reacted with ε-caprolactone.
A GPC analysis of a tetrahydrofuran eluate of the product revealed that the product has a number-average molecular weight (Mn) of 433, a weight-average molecular weight (Mw) of 563, and a ratio Mw/Mn of 1.30 in terms of polyethylene glycol. The product has an acid value of 195.2 mg of KOH per gram and a hydroxyl value of 3.3 mg of KOH per gram. The ¹H-NMR spectrum of the product is shown in FIG. 7.

### Comparative Example 1

In a glass flask equipped with a cooling tube, an exhaust vent, a nitrogen inlet tube, a thermometer, and a stirrer were placed 400.0 g (5.55 mol) of acrylic acid, 0.442 g (3.56 mmol) of MEHQ, and 30.1 g (0.158 mol) of p-toluenesulfonic acid monohydrate. After elevating the inner temperature to 80°C, 507.0 g (4. 44 mol) of ε-caprolactone was added dropwise over four hours with stirring at 80°C. The mixture was stirred for further two hours while keeping the reaction temperature to 80°C. After cooling to 30°C, the reaction mixture was combined with 6.65 g (0.166 mol) of a 10% aqueous solution of NaOH. The reaction mixture was then combined with 1.3 liters of toluene, washed with three portions of 0.9 liter of pure water, and the solution was concentrated under reduced pressure in a rotary evaporator to remove low boiling components. As a result, 1651. 7 g of an oily reaction product was obtained. A ¹H-NMR analysis revealed that 3.09 moles of ε-caprolactone on average is added to 1 mole of acrylic acid, and that only 24.5% of the charged acrylic acid reacted with ε-caprolactone.

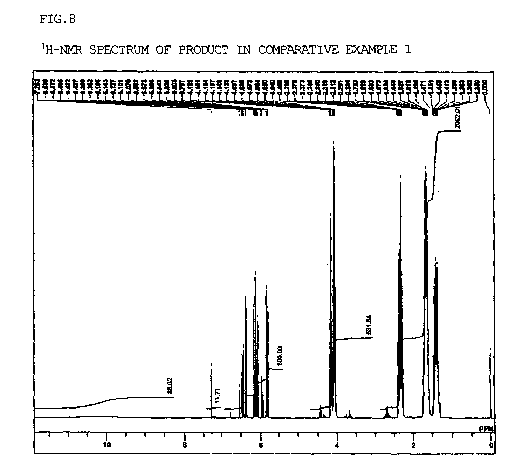

A GPC analysis of a tetrahydrofuran eluate of the product revealed that the product has a number-average molecular weight (Mn) of 569, a weight-average molecular weight (Mw) of 842, and a ratio Mw/Mn of 1.48 in terms of polyethylene glycol. The product has an acid value of 150.6 mg of KOH per gram and a hydroxyl value of 4.0 mg of KOH per gram. The ¹H-NMR spectrum of the product is shown in FIG. 8.

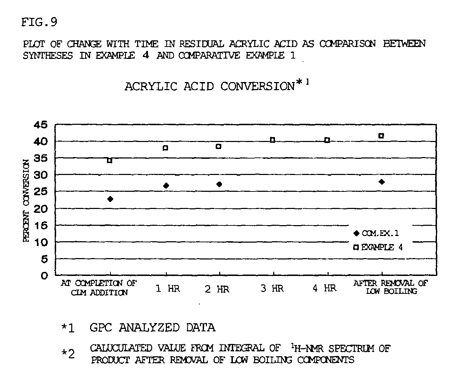

FIG. 9 shows the conversions from acrylic acid in the reaction products in Example 4 and Comparative Example 1. FIG. 9 demonstrates that the conversion from acrylic acid in Example 4 is 10% or more higher than that in Comparative Example 1 after the completion of addition of ε-acrylic acid, 2 hours into the reaction, and after removal (reaction) of low boiling components.

Table 1 shows the properties of the products after neutralization of the catalyst with an alkali, washing with water, and drying in Example 4 and Comparative Example 1. Table 1 demonstrates that, even though the ratio of acrylic acid to ε-caprolactone is the same, the product of Example 4 shows a significantly increased conversion from acrylic acid of about 1.5 times and has a lower molecular weight with a lower number of moles of added caprolactone of about two-thirds those in the conventional technique Comparative Example 1. In Example 4, a sufficient amount of water was in coexistence in the reaction.

**Table 1**

| | Water in reaction*1 | Conversion from acrylic acid*2 | Number of moles of added caprolactone |
|---|---|---|---|
| Com. Ex. 1 | 0.3 part by weight | 24.5% | 3.09 |
| Example 4 | 3.4 parts by weight | 36.7% | 2.03 |

| | | | |
|---|---|---|---|
| *1: The amount relative to 100 parts by weight of the total of acrylic acid and ε-caprolactone | | | |
| *2: The value calculated from the weight of charged acrylic acid and the weight of acrylic acid units combined with lactone in the reaction product as calculated from the integral of ¹H-NMR spectrum. | | | |

### Industrial Applicability

According to the present invention, unsaturated lactone-derived polyester monomers having a terminal carboxyl group with practical quality can be produced at low cost.

## Claims

1. A method for producing an unsaturated lactone-derived polyester monomer of Structural Formula (1): 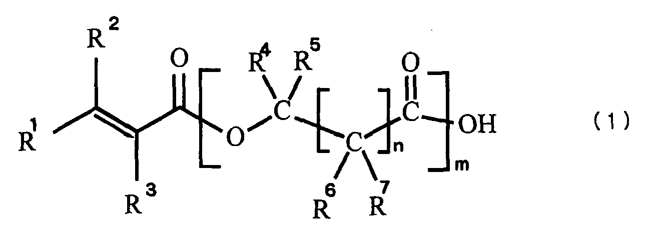
wherein each of R¹, R², R³, R⁴, R⁵, R⁶, and R⁷ represents a substituent selected from the group consisting of hydrogen atom, a substituted or unsubstituted alkyl group having one to ten carbon atoms, a substituted or unsubstituted aryl group, a substituted or unsubstituted alkoxycarbonyl group, a substituted or unsubstituted alkoxy group, a substituted or unsubstituted aryloxy group, and a halogen atom, wherein R¹, R², R³, R⁴, R⁵, R⁶, and R⁷ may be combined to form one or more rings; n denotes an integer from 1 to 7; and m denotes an integer from 1 to 100, the method comprising the steps of reacting a carboxyl-containing radically polymerizable unsaturated monomer with a cyclic lactone by the catalysis of an acidic catalyst in the presence of 1 to 50 parts by weight of water to 100 parts by weight of the total of the carboxyl-containing radically polymerizable unsaturated monomer and the cyclic lactone; and carrying out dehydration under reduced pressure for removing low boiling components to form an ester bond between a by-produced water-initiated lactone oligomer and the carboxyl-containing radically polymerizable unsaturated monomer to thereby reduce a hydroxyl value to 5.0 mg of KOH per gram or less.

2. The method for producing an unsaturated lactone-derived polyester monomer according to claim 1, wherein the carboxyl-containing radically polymerizable unsaturated monomer is at least one selected from the group consisting of acrylic acid, methacrylic acid, maleic acid, and itaconic acid.

3. The method for producing an unsaturated lactone-derived polyester monomer according to one of claims 1 and 2, wherein the cyclic lactone is at least one selected from the group consisting of ε-caprolactone, trimethyl-ε-caprolactone, monomethyl-ε-caprolactone, γ-butyrolactone, γ-valerolactone, and δ-valerolactone.

4. The method for producing an unsaturated lactone-derived polyester monomer according to any one of claims 1 to 3, further comprising carrying out dehydration under reduced pressure for removing low boiling components in the presence of residual carboxyl-containing radically polymerizable unsaturated monomer to form an ester bond between the carboxyl-containing radically polymerizable unsaturated monomer and the water-initiated lactone oligomer as a by-produced to thereby reduce the hydroxyl value to 5.0 mg of KOH per gram or less.
